(19) Europäisches Patentamt
European Patent Office
Office européen des brevets

(11) **EP 3 736 322 A1**

(12) **EUROPEAN PATENT APPLICATION**
published in accordance with Art. 153(4) EPC

(43) Date of publication:
**11.11.2020 Bulletin 2020/46**

(21) Application number: **19810576.9**

(22) Date of filing: **30.05.2019**

(51) Int Cl.:
*C12M 1/00* *(2006.01)*     *C12M 1/10* *(2006.01)*

(86) International application number:
**PCT/JP2019/021642**

(87) International publication number:
**WO 2019/230922 (05.12.2019 Gazette 2019/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.05.2018 JP 2018103357**
**29.08.2018 JP 2018159890**

(71) Applicant: **Terumo Kabushiki Kaisha**
**Tokyo 151-0072 (JP)**

(72) Inventors:
• **JINNO, Makoto**
**Tokyo 154-8515 (JP)**
• **NONOYAMA, Ryosuke**
**Tokyo 154-8515 (JP)**
• **YORI, Kouichirou**
**Ashigarakami-gun, Kanagawa 259-0151 (JP)**
• **SAMESHIMA, Tadashi**
**Ashigarakami-gun, Kanagawa 259-0151 (JP)**

(74) Representative: **Prüfer & Partner mbB**
**Patentanwälte · Rechtsanwälte**
**Sohnckestraße 12**
**81479 München (DE)**

(54) **CELL PROCESSING METHOD, DEVICE, AND SYSTEM**

(57) An object of the present invention is to realize a cell processing method, a device, and a system that has a simple constitution and are reasonable. A robot system is provided for injecting liquid by rotating an injection container in which the liquid is accommodated and an injection volume of the liquid is constant around an axis vertical to a long axis of the injection container. The robot system executes an injection start control to rotate the injection container around a predetermined axis, an injection control to stop rotation for a predetermined time and inject the liquid, and an injection end control to reversely rotate the injection container around the predetermined axis, and the predetermined time is calculated based on an injection flow rate Q [ml/s] measured in real time.

**Description**

Technical Field

The present invention relates to a cell processing method, a device, and a system.

Background Art

In recent years, an attempt has been made to transplant various types of cells for the purpose of repairing damaged tissues or the like. For example, an attempt has been made to utilize fetal cardiomyocyte(s), myoblast cell(s), mesenchymal stem cell(s), cardiac stem cell(s), ES cell(s), cardiomyocyte(s) and the like for the purpose of repairing myocardial tissue(s) damaged by ischemic heart diseases such as angina pectoris and myocardial infarction. As one of such attempts, development of a cell construct formed utilizing a scaffold or a sheet-shaped cell culture in which cells are formed into a sheet-shape has been made.

Conventionally, such a cell culture has been manually produced in a clean room called a cell processing center (CPC) by a worker who possesses deep expert knowledge. The costs and labors required for producing such a cell culture are large, and improvement of the efficiency thereof has been therefore desired. In light of these circumstances, an automatic cell culture apparatus has been proposed that performs works related to culture of these cells by an articulated robot (Patent Literature 1). However, there is a problem that it is difficult to automate high-level works which depends on the technique of the worker in cell culture.

In cell culture, a medium exchange process including a liquid-discarding work and an injection work is a process that depends on the technique of the worker. For example, the injection work includes high level of works such as a suction and injection work of a culture solution with a pipette, a work of wiping a dripping culture solution, and a pipette exchange work. In particular, when a culture solution in an injection bottle is sucked and injected into a culture flask or the like with a pipette, there is a problem that complicated pipette operation takes time and the risk of dripping from the tip of the pipette is high.

Citation List

Patent Literature

Patent Literature 1: WO 2016/104666 A

Summary of Invention

Technical Problem

Under such circumstances, in the development of means for efficient cell processing, the present inventors confronted problems, particularly, difficulty in efficiently and quickly performing an injection work which depends on a high level of technique by the worker. Therefore, an object of the present invention is to solve such problems and realize a cell processing method, a device, and a system that have a simple structure and are reasonable.

Solution to Problem

In a diligent research to solve the above-described problems, the present inventors focused on generation of variation in dripping or the injection volume when the injection work is quickly performed. As a result of the further research, the present inventors found that the above-described problems can be solved by utilizing means for restricting injection and completed the present invention.

That is, the present invention relates to the following.

[1] A device mounted on an injection container for use, the device including a cap that can be detachably attached to the injection container, an inlet tube that can be fitted in a first through hole provided in the cap, and a suction tube that can be fitted in a second through hole provided in the cap.

[2] The device according to [1], in which, the device is configured so that, in a state in which the device is mounted on the injection container, the lower end of the suction tube protruded from the cap into the injection container is disposed close to the cap.

[3] The device according to [1] or [2], in which a check valve is provided in the suction tube.

[4] The device according to any of [1] to [3], in which the first through hole is provided in the peripheral part of the cap.

[5] A method for injecting liquid, the method including (a) preparing an injection container accommodating the liquid; (b) mounting, on the injection container, a device including a cap that can be detachably attached to the injection container, an inlet tube that can be fitted in a first through hole provided in the cap, and a suction tube that can be fitted in a second through hole provided in the cap; (c) rotating the injection container to inject the liquid via the inlet tube; and (d) reversely rotating the injection container to end injection.

[6] The method according to [5], further including (e) continuously performing the injection work a plurality of times by repeating (c) and (d).

[7] The method according to [5] or [6], further including (f) determining the injection volume from the injection time, after (c) and before (d).

[8] A device mounted on an injection container for use, the device including a cap that can be detachably attached to the injection container, an inlet tube that can be fitted in a first through hole provided in the cap, and a suction tube that can be fitted in a second through hole provided in the cap, in which, the device is configured so that, in a state in which the device is mounted on the injection container, the lower end of the suction tube protruded from the cap into the injection container is disposed close to the cap.

[9] The device according to [8], in which a check valve is provided in the suction tube.

[10] The device according to [8] or [9], in which the first through hole is provided in the peripheral part of the cap.

[11] The device according to any of [8] to [10], in which the injection container is a container for accommodating and injecting a culture media, and injection is performed by rotating the injection container.

[12] A method for injecting a culture media, the method including (a) preparing an injection container accommodating a culture media; (b) mounting, on the injection container, a device including a cap that can be detachably attached to the injection container, an inlet tube that can be fitted in a first through hole provided in the cap, and a suction tube that can be fitted in a second through hole provided in the cap; (c) rotating the injection container to inject the culture media via the inlet tube; and (d) reversely rotating the injection container to end injection.

[13] The method according to [12], further including (e) continuously performing the injection work a plurality of times by repeating (c) and (d).

[14] The method according to [12] or [13], further including (f) determining the injection volume after (c) and before (d).

[15] A robot system for injecting liquid by rotating an injection container accommodating the liquid around an axis vertical to the long axis of the injection container, in which the robot system executes an injection start control to rotate the injection container around a predetermined axis, an injection control to stop rotation for a predetermined time and inject liquid, and an injection end control to reversely rotate the injection container around the predetermined axis; a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at the tip end of the inlet tube.

[16] The robot system according to [15], in which a coordinate system (TCP) for controlling a position or attitude of the robot system is set on a predetermined axis.

[17] The robot system according to [15] or [16], in which the rotation of the injection container is performed via a cap held by an end effector of the robot system.

[18] The robot system according to any of [15] to [17], in which, the robot system is configured so that, in a state in which the device is mounted on the injection container, the lower end of the suction tube protruded from the cap into the injection container is disposed close to the cap.

[19] The robot system according to any of [15] to [18], in which injection is performed on a cell culture flask, the predetermined axis and the culture surface of the cell culture flask are parallel, and the cell culture flask is obliquely disposed with the culture surface faced upward.

[20] A method for injecting liquid by rotating an injection container accommodating the liquid around an axis vertical to the long axis of the injection container, the method including: an injection start step of rotating the injection container around a predetermined axis; an injection step of stopping rotation for a predetermined time and injecting the liquid; and an injection end step of reversely rotating the injection container around the predetermined axis, in which a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at the tip end of the inlet tube.

[21] A program for controlling a robot for injecting liquid by rotating an injection container accommodating liquid around an axis vertical to the long axis of the injection container, in which the program causes a computer to execute an injection start control to rotate the injection container around a predetermined axis, an injection control to stop rotation for a predetermined time and inject the liquid, and an injection end control to reversely rotate the injection container around the predetermined axis; a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at the tip end of the inlet tube.

[22] A robot system for injecting liquid by rotating an injection container in which the liquid is accommodated and the injection volume of the liquid is constant around an axis vertical to the long axis of the injection container, in which the robot system executes an injection start control to rotate the injection container around a predetermined axis, an injection control to stop rotation for a predetermined time and inject the liquid, and an injection end control to reversely rotate the injection container around the predetermined axis, and the predetermined time is calculated based on an injection flow rate Q [ml/s] measured in real time.

[23] The system according to [22], in which the predetermined time is further calculated based on time $\Delta T$ until liquid is not injected in the reverse rotation.

[24] The system according to [22] or [23], in which the predetermined time is further calculated based on a final injection ratio X% of a case where the injection end step is further performed relative to the injection volume in a case where the injection end step is not performed.

[25] The system according to any of [22] to [24], in which a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at the tip end of the inlet tube.

[26] The system according to [25], in which, the system is configured so that, in a state in which the device is mounted on the injection container, the lower end of the suction tube protruded from the cap into the injection container is disposed close to the cap.

[27] The system according to any of [22] to [26], in which the system is configured to delay a start time of the injection end control with decrease in a remaining amount of the liquid in the injection container.

[28] A method for injecting liquid by rotating an injection container in which the liquid is accommodated and the injection volume of liquid is constant around an axis vertical to the long axis of the injection container, the method including: an injection start step of rotating the injection container around a predetermined axis; an injection step of stopping rotation for a predetermined time and injecting the liquid; and an injection end step of reversely rotating the injection container around the predetermined axis, in which the predetermined time is calculated based on an injection flow rate Q [ml/s] measured in real time.

[29] A program for controlling a robot for injecting liquid by rotating an injection container in which the liquid is accommodated and the injection volume of liquid is constant around an axis vertical to the long axis of the injection container, in which the program causes a computer to execute an injection start control to rotate the injection container around a predetermined axis, an injection control to stop rotation for a predetermined time and inject the liquid, and an injection end control to reversely rotate the injection container around the predetermined axis, and the predetermined time is calculated based on an injection flow rate Q [ml/s] measured in real time.

Advantageous Effects of Invention

According to the present invention, an efficient and quick injection work with high accuracy is possible and generation of dripping can be prevented by using means for restricting the injection. Thus, it is suitable for production of cell cultures in a clean room or the like.

Brief Description of Drawings

Fig. 1 is a schematic view of a device 1 according to the first embodiment of the present invention.
Fig. 2 is a schematic view for explaining an injection work using the device 1 of Fig.1.
Fig. 3 is a conceptual view for explaining the disposition of an accommodation container 10.
Fig. 4 is a conceptual view of a robot system according to the second embodiment of the present invention.
Fig. 5 is a schematic view for explaining a motion of the robot system of Fig. 4.
Fig. 6 is a conceptual view of a robot system according to the third embodiment.
Fig. 7 is a flow diagram of a medium exchange process.
Fig. 8 is a graph showing the injection volume and time during an injection motion.
Fig. 9 is a graph showing the injection velocity and time during an injection motion.
Fig. 10 is a method of predicting the injection volume after starting an injection end motion.
Fig. 11 is a flow diagram of introduction of parameters and a validation test.
Fig. 12 is a prediction flow chart of an injection end motion start time.
Fig. 13 is a graph showing the injection volume and time during an injection work.

Fig. 14 is the result of a validation test of an injection work.
Fig. 15 shows a suction tube and inlet tube manufactured.
Fig. 16 is a graph showing the inner diameter of a suction tube, injection velocity, and time.
Fig. 17 is a graph showing the inner diameter of an inlet tube, injection velocity, and time.
Fig. 18 is a graph showing the injection volume and time during an injection work.
Fig. 19 is a graph showing the inner diameter of an inlet tube and injection volume.
Fig. 20 shows the relationship between the length of an inlet tube and injection time.
Fig. 21 is a graph showing the injection volume and difference from a target value.
Fig. 22 is a graph showing the injection volume and injection accuracy.
Fig. 23 is a graph showing the remaining amount at start of an injection end motion and difference from a target value.

Description of Embodiments

Examples of the component constituting liquid in the present invention include water, saline, a physiological buffer (for example, HBSS, PBS, EBSS, Hepes, and sodium bicarbonate), a culture media (for example, DMEM, MEM, F12, DMEM/F12, DME, RPMI1640, MCDB, L15, SkBM, RITC80-7, and IMDM), a sugar solution (for example, a sucrose solution, and Ficoll-paque (registered trademark) PLUS), seawater, a serum-containing solution, a Renografin (registered trademark) solution, a metrizamide solution, a meglumine solution, glycerin, ethylene glycol, ammonia, benzene, toluene, acetone, ethyl alcohol, benzole, oil, mineral oil, animal fat, vegetable oil, olive oil, a colloidal solution, liquid paraffin, turpentine oil, linseed oil, and castor oil.

The accommodation container in the present invention is not particularly limited, and examples thereof include cell culture containers, cell culture flasks for adhesion cells, and cell culture flasks for floating cells. The cell culture flask refers to a container that has a substantially rectangular main body having flat surfaces, and at least one of the flat surfaces is subjected to surface treatment necessary for cell culture. The cell culture enables a multistage culture by stacking a plurality of cell culture flasks with the cell culture surface faced downward.

The injection container in the present invention is not particularly limited as long as it is a container that can accommodate, for example, a culture media to be injected in the accommodation container. Examples thereof include a shaker flask, an erlenmeyer flask, a roller bottle, an injection bottle, a beaker, a medium bottle, a square type medium bottle, a sterilized jar, and a sterilized bottle.

The robot in the present invention is not particularly limited, and examples thereof include linear motion-rotation apparatuses, manipulators, and articulated robots. Examples of the articulated robot include two-axis articulated robots, three-axis articulated robots, four-axis articulated robots, five-axis articulated robots, six-axis articulated robots, and seven-axis articulated robot.

In the present invention, the term "predetermined axis" refers to an axis that is the center of the rotation when rotating an injection container. In a case where the injection container is a common vertically long container, the predetermined axis is set as an axis vertical to the long axis of the container.

In the present invention, the term "TCP" refers to a tool center point, and means a coordinate system for expressing the position and attitude of an object to be controlled such as a tool and gripper, which are positioned at the tip end of the robot, and an object to be worked. The TCP can be set to an arbitrary position and attitude (motion, position reasonable for control, and attitude) of an end effector (for example, a gripper and a tool), an object to be worked (for example, a flask and a bottle) or the like. In the case of the six-axis articulated robot, the TCP is typically defined for the coordinate system of the sixth axis of the robot.

In the present invention, the phrase "rotating around a predetermined axis" means rotating an object around a predetermined axis. For example, when the predetermined axis is set at one end of the opening of the injection container, liquid in the injection container can be discharged by only a rotation motion around such one end. For example, even in a case where the predetermined axis is set to the center (center of gravity) of the injection container, the injection container can be rotated around one end of the opening of the injection container as described above by combining the rotation motion around the center axis of the injection container and the translation motion along the circular path.

In addition, in a case where the robot is an articulated robot for example, efficiency of the robot control can be improved by making the predetermined axis and the TCP correspond to each other. In a case where the robot is, for example, a six-axis articulated robot, when the rotation axis of the sixth axis is made parallel to the rotation axis of the TCP, the injection container can be rotated as described above by a rotation motion of the sixth axis and a slight motion of the first to fifth axis. Further, in a case where the rotation axis of the sixth axis and the rotation axis of the TCP are aligned with each other, the injection container can be rotated as described above by only the rotation motion of the sixth axis.

Examples of the cell in the present invention include, but are not limited to, adhesion cells (adhesive cells). Examples of the adhesion cell include adhesion somatic cell(s) (for example, cardiomyocyte(s), fibroblast cell(s), epithelial cell(s), endothelial cell(s), hepatic cell(s), pancreatic cell(s), renal cell(s), adrenal cell(s), periodontal ligament cell(s), gingival cell(s), periosteal cell(s), skin cell(s), synoviocyte(s), and chondrocyte(s)) and stem cells (for example, myogenic cell(s), tissue stem cell(s) such as cardiac stem cell(s), embryonic stem cell(s), and pluripotent stem cell(s) such as induced pluripotent stem (iPS) cell(s), and mesenchymal stem cell(s)). Somatic cells may also be differentiated from stem cells, particularly iPS cells. Non-limited examples of a cell that can form a sheet-shaped cell culture include myogenic cell(s) (for example, myoblast cell(s)), mesenchymal stem cell(s) (for example, cells derived from bone marrow, fat tissue, peripheral blood, skin, hair root, muscle tissue, endometrium, placenta, and cord blood), cardiomyocyte(s), fibroblast cell(s), cardiac stem cell(s), embryonic stem cell(s), iPS cell(s), synoviocyte(s), chondrocyte(s), epithelial cell(s) (for example, oral mucosal epithelial cell(s), retinal pigment epithelial cell(s), and nasal epithelial cell(s)), endothelial cell(s) (for example, vascular endothelial cell(s)), hepatic cell(s) (for example, hepatic parenchymal cell(s)), pancreatic cell(s) (for example, pancreatic islet cell(s)), renal cell(s), adrenal cell(s), periodontal ligament cell(s), gingival cell(s), periosteal cell(s), and skin cell(s). In the present invention, cell(s) that form a monolayer cell culture, for example, myogenic cell(s) or cardiomyocyte(s) are preferred, and skeletal myoblast cell(s) or cardiomyocyte(s) derived from iPS cell(s) are particularly preferred.

Hereinafter, preferred embodiments of the present invention will be described in detail with reference to the drawings.

[First embodiment]

An aspect of the present invention is a device mounted on an injection container for use, the device including a cap that can be detachably attached to the injection container, an inlet tube that can be fitted in a first through hole provided in the cap, and a suction tube that can be fitted in a second through hole provided in the cap.

Firstly, the first embodiment of the present invention will be described.

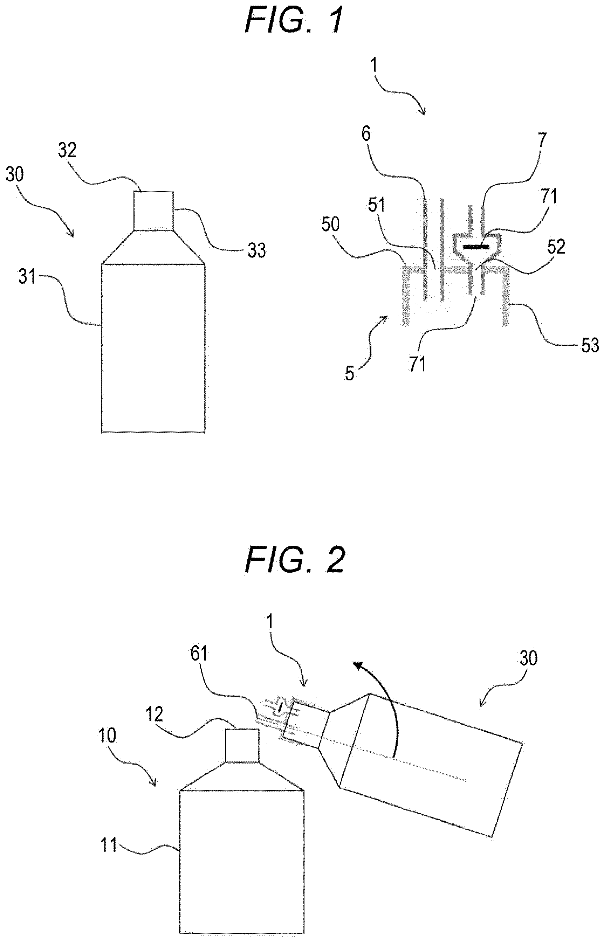

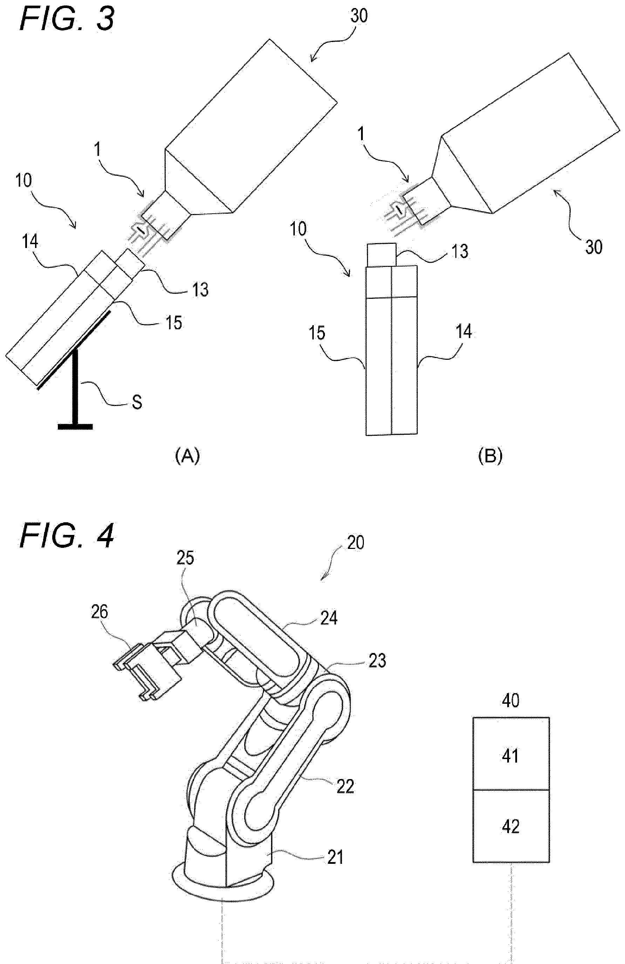

Fig. 1 is a schematic view of a device 1 according to the first embodiment of the present invention. Fig. 2 is a schematic view for explaining an injection work using the device 1 of Fig.1. Fig. 3 is a conceptual view for explaining the disposition of an accommodation container 10. In the present embodiment, description will be made on the assumption that liquid is a culture media; an injection container 30 is an injection bottle to accommodate the culture media; an accommodation container 10 is a cell culture flask; and an injection work is performed in a clean room.

Note that, in each drawing in the present application, the size of each component is appropriately emphasized for ease of explanation, and the size of each component shown does not indicate the actual size.

As shown in Fig. 1, a device 1 according to the first embodiment of the present invention includes a cap 5 that can be detachably attached to a mouth portion 33 of an injection container 30 that accommodates a culture media and has an opening 32, an inlet tube 6 that can be fitted in a first through hole 51 provided in a top plate 50 of the cap 5, and a suction tube 7 that can be fitted in a second through hole 52 provided in the top plate 50 of the cap 5. The top plate 50 has a disk shape that can cover the opening 32 of the injection container 30 and has a cylindrical skirt wall 53 pending from the peripheral part. The inner peripheral surface of the cylindrical skirt wall 53 is provided with an inner thread (not shown), and the inner screw can be screwed into an outer screw (not shown) provided in the outer peripheral surface of the mouth portion 33 of the injection container 30.

The first through hole 51 is disposed around the peripheral part of the top plate 50. The inlet tube 6, which is fitted in the first through hole 51, is disposed around the inner peripheral surface of the mouth portion 33 of the injection container 30 in a state in which the cap 5 is attached to the injection container 30. The first through hole 51 is configured such that when the injection container 30 is tilted to discharge a culture media, liquid is discharged without being left in the injection container 30. The length of the inlet tube 6 is not particularly limited, preferably 0 to 100 mm, more preferably 20 mm to 70 mm, and even more preferably 30 to 60 mm. The inner diameter of the inlet tube 6 is preferably 1 to 10 mm, and more preferably 3 to 5 mm. The length of a protruded portion of the inlet tube 6, protruded from the top plate 50 when the inlet tube 6 is fitted in the first through hole 51 is not particularly limited, but can be set to preferably 0 to 100 mm, and more preferably 10 to 40 mm. The flow rate (flow volume per unit time) of the inlet tube 6 is 1.0 ml/s to 20ml/s, preferably 2.0 ml/s to 15 ml/s, and even more preferably 2.5 ml/s to 10.3 ml/s. The combination of the inner diameter and the flow rate of the inlet tube 6 can be set so that when the inner diameter is in a range of 3 mm to 5 mm, the flow rate is in a range of 2.0 ml/s to 10.0 ml/s. Further, assuming that the cross-sectional area of the inner diameter is proportional to the flow rate, setting can be possible such that when the inner diameter is in a range of 6 mm to 10 mm, the flow rate is in a range of approximately 15 ml/s to 40 ml/s. The length, inner diameter, and flow rate of the inlet tube can be freely selected and combined as long as the flow volume per unit time is constant.

The length of the suction tube 7 is not particularly limited, preferably 0 to 100 mm, and more preferably 10 mm to 60 mm. The inner diameter of the suction tube 7 is preferably 0.5 to 10 mm, and more preferably 2 to 4 mm. Also, the suction tube 7 has preferably a smaller diameter compared to the inlet tube. The length of a protruded part of the suction tube 7, protruded from the top plate 50 when the suction tube 7 is fitted in the second through hole 52 is not particularly limited as long as the length is such that the lower end 71 of the suction tube 7 is disposed near the top plate 50 of the cap 5, but can be set to preferably 0 to 100 mm, and more preferably 0 to 40 mm. The suction tube 7 is provided with a check valve 71, and the check valve 71 is configured to allow air from the outside of the injection container 30 to pass through but does not allow a culture media from the inside of the injection container 30 to pass through. Note that the check valve may also be disposed inside the injection container 30. Note that the inlet tube 6 and the suction tube 7 may be integrally formed with the cap 5.

As shown in Fig. 2, when the device 1 is used, the device 1 is attached to the injection container 30 accommodating a culture media, and the injection container 30 is tilted to determine the position of the tip end 61 of the inlet tube 6 at an injection container 30 side of the opening 12 of the accommodation container 10. At this time, it is preferred to configure when the injection container 30 is tilted, liquid moves not to the suction tube 7 side, but to the inlet tube 6 side by rotating and positioning such that the inlet tube 6 (first through hole) is positioned lower than the suction tube 7 (second through hole). Then, the injection container 30 is rotated around the tip end 61 of the inlet tube 6 in the arrow direction to transfer the culture media inside the injection container 30 to the device 1 side (injection start motion). When the rotation is stopped for a predetermined time in a state in which the opening 32 of the injection container 30 is positioned at a lower side, the culture media is discharged from the inlet tube 6 and injected into the accommodation container 10 (injection motion). When the injection volume in the accommodation container 10 reaches a predetermined amount, the injection container 30 is reversely rotated in a direction reverse to the arrow direction and stopped to transfer the culture media to a side opposite to the device 1, thus ending the injection (injection end motion).

In the injection motion, the injection volume of the culture media discharged (injected) from the inlet tube 6 and the suction amount of air which flows in from the suction tube 7 are equivalent. Since the injection volume and the suction amount are restricted by the dimension of the inlet tube 6, the dimension of the suction tube 7 or the like, the injection volume is restricted compared to the case of not using the tube (means for restricting injection). Further, when the air flowed into the injection container 30 becomes continuous bubbles and the suction amount per unit time becomes constant, the injection volume per unit time also becomes constant. In this case, since the injection time and the injection volume in the injection work are proportional to each other, an injection work with high accuracy can be performed by measuring the relationship between the injection volume and the time required for the injection in advance and determining the injection volume from the injection time based on the measured result.

In general, in a case where air inside the injection container 30 is released to the atmospheric pressure, as the amount of the culture media in the injection container 30 is large, the injection volume per unit time is large. In the case of the configuration of the present invention, air inside the injection container 30 is not released to the atmospheric pressure at the time of injection, and thus the pressure inside the injection container 30 is a negative pressure. Since the larger the amount of the culture media in the injection container 30 is, the larger the negative pressure of the air inside the injection container 30 is, the injection volume per unit time becomes small compared to the case where air inside the injection container 30 is released to the atmospheric pressure. By continuously incorporating air into the injection container 30 in association with the injection, the air pressure inside the injection container 30 becomes gradually close to the atmospheric pressure. The injection volume per unit time is constant due to these effects.

Conventionally, in a case where 75 ml of a culture media is injected into a culture flask for example, the following complicated works need to be repeated: a pipette is inserted into the injection bottle; a culture media is sucked; a quantity of 75 ml is visually confirmed; and the pipette is moved to inject the culture media into the culture flask. On the other hand, when the device 1 of the present invention is used, for example, a device 1 is attached to the injection container 30 accommodating approximately 480 ml of a culture media; an injection motion is performed for only the time required for injection of 75 ml measured in advance; and this injection motion can be continuously (six times) performed by exchanging six accommodation containers 10. Thus, the conventional complicated work can be replaced with a simple work which is the repetition of the rotation motion of the injection container 30. As a result, operation of a pipette, transferring, visual confirmation of the quantity, and the like are eliminated, whereby time related to the injection work can be remarkably reduced.

Also, conventionally, in a case where a pipette is used, suction and discharge of the culture media is performed via one flow path, which causes dripping due to misoperation. Meanwhile, by using the device of the present invention, the suction work of the culture media is eliminated and the discharge of the culture media and the suction of air are performed in separate flow paths, so that generation of dripping can be minimized. Further, since injection of the culture media is performed via a tube by using the device of the present invention, the injection direction and the injection range of the culture media is restricted by the protrusion direction and the aperture of the tube (means for restricting injection). As a result, the accuracy of the sight of the injection is enhanced, for example, an injection work can be performed by accurately focusing on an accommodation container having a small opening such as an opening of a cell culture flask, whereby generation of dripping can be minimized.

Then, the disposition of the accommodation container 10 to inject the culture media can take various forms. For example, in a case where the accommodation container 10 is a cell culture flask, the accommodation container 10 is preferably disposed so that main surfaces 14 and 15 are parallel to the rotation axis as shown in Fig. 3A. In addition, the accommodation container 10 may be placed on a base S or the like, and obliquely disposed with the main surface 14 (culture surface) of the accommodation container 10 faced upward. As a result of this, the culture media discharged from the inlet tube 6 flows in along the inclined surface, and thereby bubbling is less likely to occur. Similarly, as shown in Fig. 3B, the accommodation container 10 may also be disposed upright so that the main surface 14 (culture surface) of the injection container 30 faces the proximal side and the main surface 15 side faces the distal side. With this, the culture media flows in along a portion with a small step between one side of the mouth portion 13 and the main surface 15, so that not only bubbling is less likely to occur, but also the culture media is not in direct contact with the main surface 14 (culture surface).

In the case of disposing the lower end 71 of the suction tube 7 close to the top plate 50 of the cap 5 as described above, bubbles are continuously generated in the culture media. However, a configuration is possible such that generation of bubbles in the injection container 30 is prevented by increasing the length of the protrusion of the suction tube 7, whereby precluding occurrence of bubbles in the culture media. That is, it is configured such that, when the injection container 30 is tilted to transfer the culture media to the device 1 side, an air layer is formed on the bottom side of the injection container 30, and the lower end 71 of the suction tube 7 is positioned at the air layer. The length of a protruded part of the suction tube 7, protruded from the top plate 50 in this case is not particularly limited as long as the lower end 71 of the suction tube 7 is disposed close to the bottom surface of the injection container 30, but can be set to preferably 0 to 100 mm, and more preferably 0 to 50 mm from the bottom of the injection container 30. Thus, an injection work with high accuracy can be performed by measuring the relationship between the injection volume and the time required for the injection in advance and determining the injection volume from the injection time based on the measured result as described above.

As described above, according to the injection device 1 of the first embodiment of the present invention, utilizing means for restricting the injection allows an efficient and quick injection work with high accuracy and also prevents generation of dripping. Thus, the injection device 1 of the first embodiment of the present invention is suitable for production of cell cultures in a clean room or the like.

[Second embodiment]

Another aspect of the present invention relates to a robot system for injecting liquid by rotating an injection container accommodating liquid around an axis vertical to the long axis of the injection container, in which the robot system executes an injection start control to rotate the injection container around a predetermined axis, an injection control to stop rotation for a predetermined time and inject the liquid, and an injection end control to reversely rotate the injection container around the predetermined axis; a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at the tip end of the inlet tube.

Then, the second embodiment of the present invention will be described.

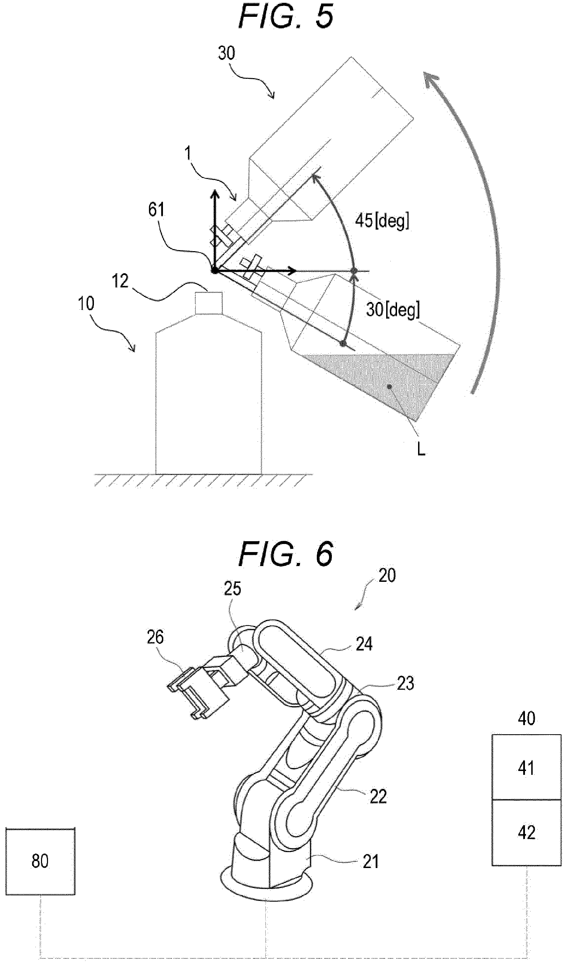

Fig. 4 is a conceptual view of a robot system according to the second embodiment of the present invention. Fig. 5 is a schematic view for explaining a motion of the robot system of Fig.4. Note that, in each drawing in the present application, the size of each component is appropriately emphasized for ease of explanation, and the size of each component shown does not indicate the actual size.

Further, in the drawings, the configurations that are the same as those of the device 1 according to the first embodiment are given the same reference signs. Hereinafter, the difference between the first embodiment will be described in detail, and the description of the same matters will be omitted.

As shown in Fig. 4, a robot 20 is a six-axis vertical articulated robot disposed on a base mount. The robot 20 includes a base 21 that can turn with respect to the base mount; a first arm 22 that is connected to the base 21 and can be tilted with respect to the vertical axis of the turning direction of the base 21; a second arm proximal end part 23 that is connected to a tip end side of the first arm 22 and can be tilted with respect to the first arm 22; a second arm distal end part 24 that is connected to the tip end side of the second arm proximal end part 23 and can rotate with respect to the axis direction of the second arm proximal end part 23; a hand part 25 that is connected to the tip end side of the second arm distal end part 24 and can be tilted with respect to the axis direction of the second arm distal end part 24; and a gripper 26 (end effector) that is connected to the hand part 25. Note that the hand part 25 is configured to be rotatable along the axis direction thereof.

Further, the robot 20 can communicate with a control device 40 including a storage unit 41 that stores a program describing the control process of the robot 20, and a processing unit 42 that processes the program to control the robot 20. The robot 20 can be automatically operated in accordance with the control signal by the control device 40. With such a configuration, the robot 20 can automatically determine the position and attitude of the gripper 26, and also rotate, open and close the gripper 26, so that transferring, tilting, rotating of the injection container 30 is automatically made possible by the gripper 26.

Hereinafter, the injection system using the above-described robot system of the present embodiment will be described. In the present embodiment, when liquid is injected into the accommodation container 10 using an injection container 30 as described in the first embodiment, the robot system is used for rotating the injection container 30 via the gripper 26 (end effector) of the robot 20, discharging the liquid, and injecting the liquid into the accommodation container 10. In the present embodiment, description will be made on the assumption that liquid is a culture media; an injection container 30 is an injection bottle to accommodate the culture media; an accommodation container 10 is a cell culture flask; and an injection work is performed in a clean room. Further, description will be made on the assumption that the cell culture flask has two main surfaces 14 and 15 as its side surface; one main surface 14 is subjected to surface processing; and cell culture can be possible with the main surface 14 facing downward.

With reference to the schematic view of Fig. 5, firstly, in a case of using a robot system, the control device 40 is started to cause the processing unit 42 to read a program stored in the storage unit 41. Based on the program, the processing unit 42 controls the robot 20 so that the gripper 26 (not shown) holds the cap 5 of the injection container 30 mounting the device 1 so as to interpose the cap 5 therein. At that time, the side of the first through hole 51 of the device 1 is made so as to face the direction of the accommodation container 10. Next, through rotation and translation motions performed by controlling the robot 20, the position of the tip end 61 of the inlet tube 6 fitted in the first through hole 51 is determined at the injection container 30 side of the opening 12 of the accommodation container 10. Then, an axis vertical to the long axis of the injection container 30 that intersects with the tip end 61 is set as a rotation axis A (position determination control).

At that time, accurate positioning may also be automatically performed by interlocking a camera (not shown) monitoring the position and angle of the accommodation container 10 and the injection container 30 with the robot 20. Thus, the robot system may also include a monitoring camera that can communicates with the control device 40, and the program may be a program that controls the robot 20 based on the position and angle monitored by the camera. The rotation axis A is set so as to be vertical to the long axis of the injection container 30, and the tip end 61 is disposed above the opening 12 of the accommodation container 10. Preferably, the distance from the opening 12 is set to a range of 0 to 3 cm.

Next, the injection container 30 to which the cap 5 is attached is rotated around the rotation axis A in the arrow direction by controlling the robot 20 (injection start control). As shown in Fig. 5, in the present embodiment, for the start angle of the injection container 30 in the injection start control, the inlet tube 6 was set 30 deg upward from the horizontal direction, and for the stop angle, the inlet tube 6 was set 45 deg downward from the horizontal direction. However, these angles are not limited thereto and, for example, the stop angle may also be set in a range of 5 to 85 deg. A configuration is possible such that foaming of the culture media is prevented by causing the culture media discharged from the tip end 61 of the inlet tube 6 to be injected obliquely to the inner wall of the mouth portion 13 of the accommodation container 10 and the inner wall of the container body 11.

Next, when rotation is stopped for a predetermined time in a state in which the mouth portion 33 of the injection container 30 is positioned in a lower side by controlling the robot 20, the culture media inside the injection container 30 moves to the cap 5 side to be discharged from the inlet tube 6 and injected into the accommodation container 10 (injection control). Then, after elapse of a predetermined time, the injection container 30 is reversely rotated in a direction reverse to the arrow direction and stopped at the start angle, to thereby move the culture media in a direction opposite to the mouth portion 33 side, thus ending the injection (injection end control). Thus, the injection work for a plurality of accommodation containers 10 can be efficiency and quickly performed by causing the robot 20 to repeatedly execute the injection start control, injection control, and injection end control while exchanging the accommodation container 10. For the above-described predetermined time, the relationship between the injection volume and the injection time is measured in advance, and time (predetermined time) to stop rotation may be set based on the measurement value and the target injection volume.

Meanwhile, when the remaining amount of the culture media in the injection container 30 becomes small, it is also possible that only the injection container 30 is removed from the cap 5 held by the gripper 26, a new injection container 30 is attached to the cap 5, and the injection start control, injection control, and injection end control are repeatedly performed by the robot 20. By repeating exchange of the accommodation container 10 and exchange of the injection container 30 as described above, the setting of the rotation axis A of the injection container 30 (position determination control) can be omitted, and thus the interruption time of the injection work can be minimized. The robot system of the present invention is only required to perform the rotation motion related to at least injection start control, injection control, and injection end control. The robot system may also be configured, for example, to repeat steps of rotation, stop, and reverse rotation by using a simple device such as a linear motion-rotation apparatus as a robot. The robot system of the present invention may also be configured such that the tip end 61 of the inlet tube 6 and the opening 12 are closely disposed during at least injection control by setting the rotation axis to the cap 5 or the injection container 30.

The disposition of the accommodation container 10 is preferably configured such that the main surfaces 14 and 15 of the accommodation container 10 (cell culture flask) are disposed so as to be parallel to the rotation axis A as described above. It may be configured such that this disposition is performed by a robot control. That is, for example, the program and the processing unit 42 may also be configured such that the disposition of the accommodation container 10 is confirmed by a camera capable of communicating with a robot system, and the positional determination of the rotation axis A is controlled according to the directions of the main surfaces 14 and 15 of the accommodation container 10 by controlling the robot 20. Further, it may be configured such that the injection volume in the accommodation container 10 is measured in real time by such a camera, and when the injection volume reaches a predetermined amount, the control is moved to the injection end control; or such that the injection volume in the accommodation container 10 is measured by weight in real time by an electronic balance capable of communicating with a robot system, and when the injection volume reaches a predetermined weight amount, the control is moved to the injection end control.

Further, the present invention is also realized by implementing the following processes. That is, it is a process that causes the robot to read a software (program) for realizing the above-described functions of the embodiment via the network or various storage media, and causes the processing unit (e.g., CPU, and MPU) built in the robot to execute the program. The present invention can also be realized by causing the above-described control device including a storage unit that stores a program, and a processing unit that processes the program to transmit a control signal to a robot and control the robot to be operated.

As described above, according to the robot system of the second embodiment of the present invention, an efficient and quick injection work with high accuracy can be possible and generation of dripping can be prevented by utilizing means for restricting injection. Thus, the robot system according to the second embodiment of the present invention is suitable for production of cell cultures in a clean room or the like.

[Third embodiment]

Another aspect of the present invention relates to a robot system for injecting liquid by rotating an injection container in which the injection volume of liquid accommodated is constant around an axis vertical to the long axis of the injection container, in which the robot system executes an injection start control to rotate the injection container around a predetermined axis, an injection control to stop rotation for a predetermined time and inject the liquid, and an injection end control to reversely rotate the injection container around the predetermined axis; the predetermined time is calculated based on the injection flow rate Q [ml/s] measured in real time.

Next, the third embodiment of the present invention will be described. Fig. 6 is a conceptual view of a robot system according to the third embodiment.

As shown in Fig. 6, the robot system in the present embodiment can communicate with an electronic balance 80. The electronic balance 80 can measure the weight of the accommodation container 10 in real time, calculate the injection volume and the injection flow rate, and send a feedback to the control device 40.

In the present embodiment, the robot system can set a predetermined time based on the injection flow rate Q [ml/s] measured in real time by the electronic balance 80 when the control is moved to the injection control to stop rotation for a predetermined time. The predetermined time is set by, for example, calculating time required for reaching the target injection volume from the slope of the injection flow rate Q [ml/s]. When the predetermined time has passed, the injection container is reversely rotated around a predetermined axis, and then the control is moved to the injection end control. As such, by configuring the robot system such that the predetermined time is set based on the injection flow rate Q [ml/s], the accuracy of the injection volume injected in the accommodation container 10 is increased.

Meanwhile, there may be a case where liquid in the inlet tube 6 is discharged (injected) during the injection end control (reverse rotation) caused by, for example, the start angle, stop angle, and rotation speed (angular velocity) in the injection start control, the reverse rotation speed in the injection end control, and the remaining amount in the injection container 30. Thus, the robot system in the present embodiment may also be configured such that the predetermined time in the injection control is set in consideration of the injection volume of liquid that can be injected during the injection end control.

For example, a series of the injection start control, injection control, and injection end control are performed in advance, and time $\Delta T$ until all the liquid in the inlet tube 6 is discharged (injected) in the injection end control (reverse rotation). Then, such time $\Delta T$ is stored in the storage unit 41, and in the actual injection control, time obtained by subtracting the time $\Delta T$ from the predetermined time calculated based on the injection flow rate Q [ml/s] as described above may also be set as the predetermined time. With this, transition from the injection control to the injection end control can be performed earlier by time $\Delta T$. As a result, even in a case where liquid in the inlet tube 6 is discharged during the injection end control, the accuracy of the injection volume injected into the accommodation container 10 can be made high.

Also, in a case where the reverse rotation speed (angular velocity) in the injection end control is late, the injection flow rate gradually becomes small, that is, the injection flow rate in time $\Delta T$ in the injection end control is not in the proportional relationship like the injection flow rate Q [ml/s], but shows a moderate curve. Accordingly, in the time $\Delta T$, there may be a difference in the injection volume in the case where the injection end control is not performed and the injection volume in the case where the injection end control is performed. The robot system in the present embodiment may also be configured such that the predetermined time in the injection control is set in consideration of such a difference.

For example, the ratio (final injection ratio X%) of the injection volume in the case where the injection end control is performed relative to the injection volume in the case where the injection end control is not performed is measured in advance and stored in the storage unit 41. In the actual injection control, the injection volume Vx [ml] after time $\Delta T$ is calculated from the injection flow rate Q [ml/s], time $\Delta T$, and final injection ratio X%. Thus, by setting, as the predetermined time, time required for reaching the injection volume Ve [ml] obtained by subtracting the injection volume Vx [ml] from the target injection volume, the accuracy of the injection volume injected into the accommodation container 10 can be made high even in a case where the difference occurs in the injection volume.

[Example]

Hereinafter, the robot system according to the third embodiment will be described in more detail with reference to Examples. However, these are specific examples of the present invention and the invention is not to be limited to this.

Example 1: injection work by manual work

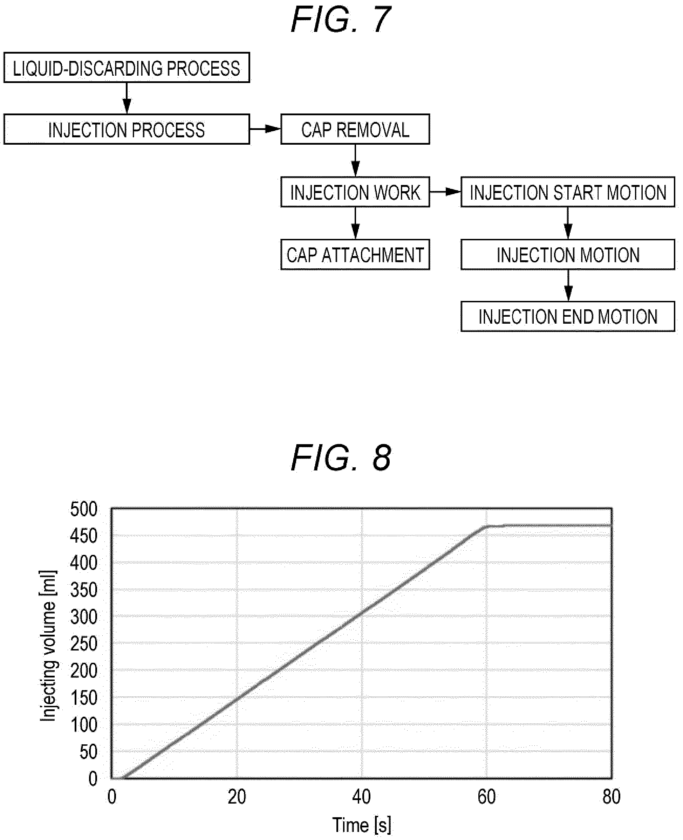

Fig. 7 is a flow diagram of a general medium exchange process. The medium exchange process is a series of works including discharging a culture solution in a flask and injecting a new culture solution in the flask. The medium exchange process must be quickly implemented without dripping, but is a work that depends on the motion based on the sense of the worker. The medium exchange process includes a liquid-discarding process and an injection process, and the injection process includes works such as removing a cap, a work of injecting a new culture solution with a pipetter, and attaching a cap. The injection process includes a work of removing and attaching the cap of the flask, a work of wiping the culture solution dripped, and a work of exchanging the pipette, in addition to a work of sucking and injecting a culture solution with a pipette.
As the accommodation container 10, a cell culture flask for adhesion cells (T500 flask, available from Thermo Fisher Scientific) was used. The outer diameter of the discharge port (opening 12) of the T500 flask was 28.2 [mm], and the inner diameter was 25.8 [mm], and the thickness was 1.2 [mm]. In the actual medium exchange work, the worker involved in the cell processing work carried out the work of sucking a culture solution in an injection bottle using an electric pipette and injecting the culture solution into a flask. 75 ml of a culture solution was each injected into eight flasks for one set. Analyses were performed on works of two sets. The result showed that the work time per one set was 550 s to 560 s, and the injection work time per flask was approximately 70 s.

Example 2: injection work using device

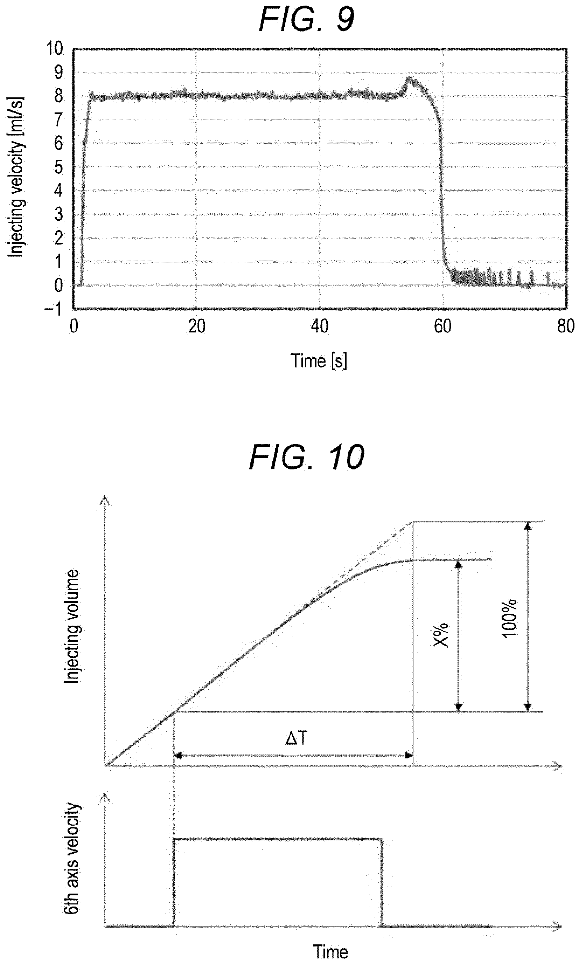

As a check valve 71, 105-15001, available from KIJIMA Co Ltd. was used; as a robot, a six-axis vertical articulated industrial robot: MOTOMAN-MH3F, Yaskawa Electric Corporation (first arm: 260 mm, second arm: 270 mm) was used; as a controller, a system using RTLinux (registered trademark) environment using as a base was used; as an electronic balance, EK-610i available from A&D Company, Limited, which can obtain a measurement value at a frequency of 100 ms was used. A device was mounted on an injection bottle, and the cap of the device was fixed to the gripper of the robot. The tip end of the inlet tube is determined as the tool center point (TCP), and an injection work to the T500 flask was performed by only the change in attitude with respect to the TCP. Further, by aligning the rotation axis of the sixth rotation axis of the robot and the TCP, the injection work by using only the sixth axis was made possible.
As shown in Fig. 5, the flask was rotated from the initial attitude where the flask is tilted by 30 deg upward from the horizontal direction to the attitude where the flask is tilted by 45 deg downward from the horizontal direction, and then rotation was stopped, thus the culture solution in the injection bottle was injected into a flask placed on the electronic balance. Figs. 8 and 9 show the injection volume (injection volume), the time, the injection velocity (injection velocity) when 480 ml of a culture solution was all injected. Since the injection velocity was approximately constant in the section excluding the injection start and the injection end (injection time: 3 to 53s, injection volume: 10 to 412 ml), it was confirmed that it was a method suitable for controlling the injection volume.

Example 3: injection work by robot

480 ml of a culture solution was divided into six and 75 ml was injected each by controlling a robot. In consideration of an error, the volume of the culture solution was set to be more than 75 ml $\times$ 6 times. The target accuracy per injection was set to $\pm 2\%$. The injection work by the robot was performed in three stages: (1) an injection start motion (control) of rotating the flask from the initial attitude where the flask was tilted upward by 30 deg from the horizontal direction to the attitude where the flask was tilted downward by 45 deg from the horizontal direction to start injection; (2) an injection motion (control) of injecting in the attitude where the flask is tilted downward by 45 deg from the horizontal direction while stopping; and (3) an injection end motion of turning the flask from the attitude where the flask is tilted downward by 45 deg from the horizontal direction to the initial attitude to end the injection.

To realize smooth motion without dripping, the injection start motion was performed at 32 deg/s, and the injection end motion was performed at 108 deg/s (angular velocity: axis velocity). For setting the injection volume for one injection to 75 ml, it was necessary to predict the injection volume after starting the injection end motion and move from the injection motion to the injection end motion. Fig. 10 shows the method for predicting the injection volume after starting the injection end motion. The time ($\Delta$T) from the time at which the injection end motion starts to the time at which the injection volume does not increase, and the final injection ratio (X%) of the injection volume in a case where the injection end control is performed relative to the injection volume in a case where the injection end control is not performed were set as a parameter. The injection volume in a case where the injection end motion is not performed was determined from the slope of the measurement value obtained during the injection motion, and the injection volume after starting the injection end motion was calculated from the obtained value. Note that the angular velocity pattern around the predetermined axis (sixth axis angular velocity pattern) is a rectangle in Fig. 10, but is not limited thereto, and may be an acceleration pattern such as a trapezoid, and an S-shape.

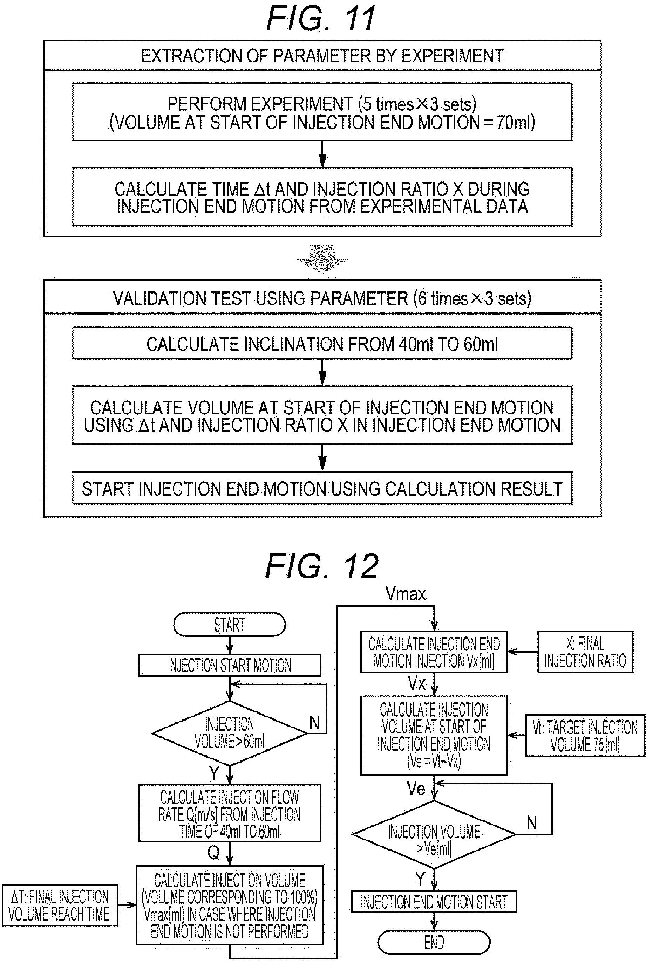

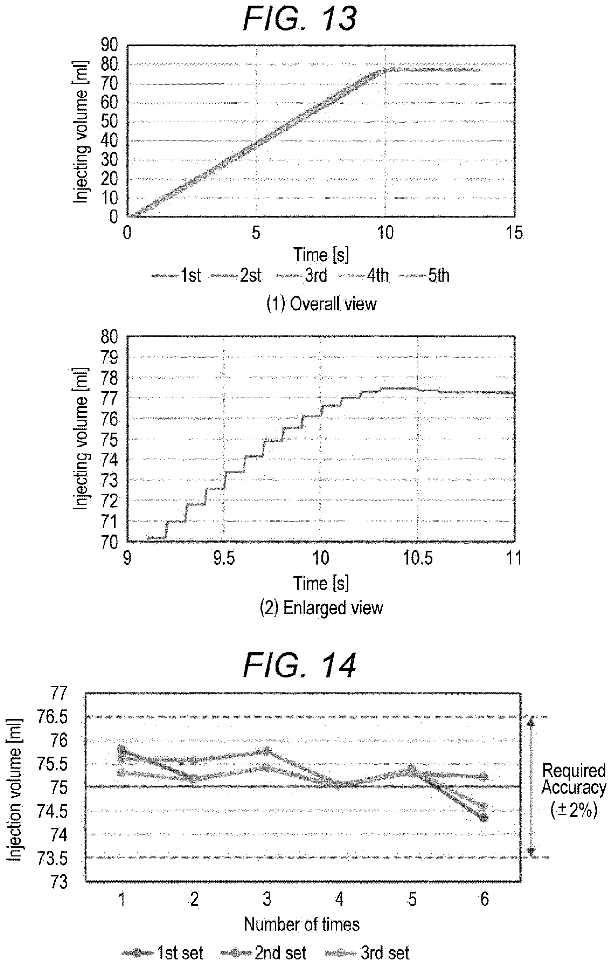

Parameter introduction and operation confirmation using parameters were performed by the procedure in Fig. 11. First, parameters were determined by analyzing data when the injection work was repeated. Assuming that the injection volume at start of the injection end motion is set to 70 ml, 3 sets of experiments were conducted where 480 ml of a culture solution was separately injected in five divided injections. Fig. 12 shows a prediction flow chart of the injection end motion start time. Fig. 13 shows the injection volume at that time. Table 1 shows analysis results.

[Table 1]

Table 1 Analysis of injection motion

| - | $\Delta$T [s] | Inclination from 40 ml to 60 ml [ml] | X [%] |
|------|------|------|------|
| Min. | 1.02 | 7.98 | 76.58 |
| Max. | 1.16 | 8.13 | 84.53 |
| Avg. | 1.07 | 8.04 | 80.88 |

As shown in Fig. 13, for the data acquired from the electronic balance, the injection volume reaches the maximum value once and then settles at the final value. The time until the injection volume firstly reaches a value after being stabilized was defined as $\Delta$T. From the experiment of Example 2, it was found that when the injection volume exceeded approximately 10 ml, the injection velocity became stable. However, since it was conceived that use of a value closer to the value of the injection end motion allows more accurate prediction, the slope for 40 ml to 60 ml was employed. The average value in Table 1 was employed as a parameter, and $\Delta$T was determined to be 1.07 s, and the injection rate in the injection end motion was determined to be 80.88%.

Then, operation confirmation was performed using the determined parameters. Fig. 14 and Table 2 show the results of three sets of experiments where 480 ml of a culture solution was divided into six and 75 ml was each injected. The injection work could be performed in a range of target accuracy $\pm$2% (required accuracy) without dripping outside the flask or on the tip end of the tube. According to the result of analysis of Example 1, the injection work time per injection in the case of the manual work was approximately 70 s. On the contrary, from the result of Fig. 13, the injection time per one injection in the case of using a robot was approximately 15 s. Since the culture solution is directly injected from the injection bottle in the technique conducted in this time, the suction work was unnecessary. In the case of combining a work by a robot and a manual work, it was confirmed that work time could be reduced to half or less even when cap removal and attachment were performed by the manual work.

[Table 2]

Table 2 Experimental result

| - | Volume [ml] | Error [%] |
|------|------|------|
| Min. | 74.34 | -0.88 |
| Max. | 75.80 | +1.07 |

(continued)

| - | Volume [ml] | Error [%] |
|------|-------------|-----------|
| Avg. | 75.25 | +0.33 |

Example 4: relationship between inner diameter of suction/inlet tube and flow rate [ml/s]

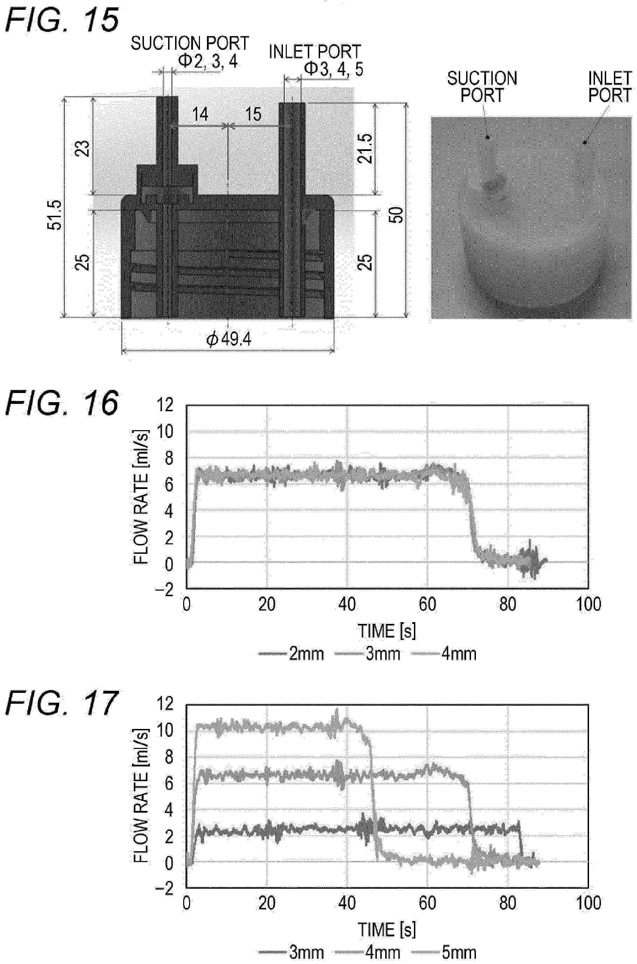

It is necessary to keep the flow rate constant for improving the injection accuracy. Thus, as a cap of the injection bottle, a cap including a suction tube (suction port) and an inlet tube (inlet port) as shown in Fig. 15 was manufactured by a 3D printer. The suction tube plays a role which is the same as a one-way valve (check valve) that allows air from the outside to pass through but does not allow a culture solution from the inside to pass through. The caps were manufactured under the following conditions; the length of the suction tube was 51.5 mm; the length of a part of the suction tube protruded from the top plate to the container direction was 25 mm; the length of a part of the suction tube protruded from the top plate to the tip end direction of the suction tube was 23 mm; the length of the inlet tube was 50 mm; the length of a part of the inlet tube protruded from the top plate to the container direction was 25 mm; the length of a part of the inlet tube protruded from the top plate to the tip end direction of the inlet tube was 21.5 mm; the inner diameter of the suction tube was changed to 2 mm, 3 mm, and 4mm respectively; and the inner diameter of the inlet tube was changed to 3 mm, 4 mm, and 5 mm respectively.

To analyze the impact of the inner diameters of the suction tube and the inlet tube on the flow rate, the flow rate when all the 480 ml of a culture solution was injected was measured by changing the diameter of each tube. Fig. 16 shows the flow rate in a case where the diameter of the inlet tube was fixed to 4 mm, and the inner diameter of the suction tube was set to 2 mm, 3 mm, and 4mm. Fig. 17 shows the flow rate in a case where the diameter of the suction tube was fixed to 3 mm, and the inner diameter of the inlet tube was 3 mm, 4 mm, and 5 mm. As a result, it was found that the inner diameter of the suction tube does not affect the flow rate, but when the inner diameter of the inlet tube increases, the flow rate increases. Tables 3 and 4 show the relationship between the inner diameters of the suction tube and inlet tube, the flow rate, and flow velocity. As the flow rate, an average value in a period in which the culture solution is stably injected in Figs. 16 and 17 was employed. The flow velocity was calculated from the flow rate and the cross-sectional area. From the result of Table 4, the flow velocity was substantially constant with the exception of the case where the inner diameter of the inlet tube is 3 mm.

[Table 3]

Table 3 Relationship between inner diameter of suction and

inlet port and flow rate [ml/s]

| Inlet port / Suction port | 3 mm | 4 mm | 5 mm |
|---------------------------|------|------|-------|
| 2 mm | – | 6.66 | – |
| 3 mm | 2.46 | 6.62 | 10.32 |
| 4 mm | – | 6.68 | – |

[Table 4]

Table 4 Relationship between inner diameter of suction and inlet port and flow velocity [m/s]

| Inlet port / Suction port | 3 mm | 4 mm | 5 mm |
|---|---|---|---|
| 2 mm | – | 0.530 | – |
| 3 mm | 0.349 | 0.527 | 0.525 |
| 4 mm | – | 0.532 | – |

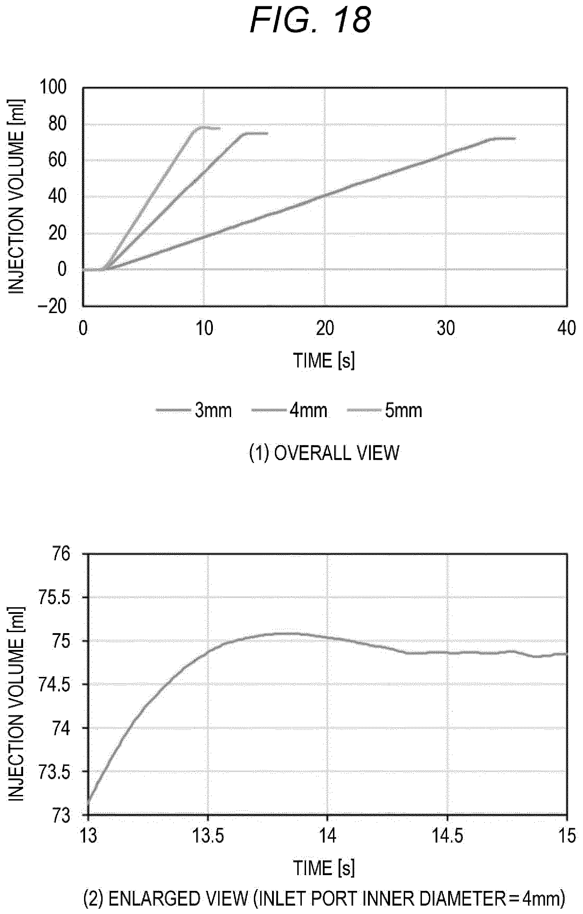

Parameter introduction and operation confirmation using parameters were performed by the procedure in Fig. 11. First, parameters were determined by analyzing data when the injection work was repeated. Assuming that the injection volume at start of the injection end motion is set to 70 ml, an experiment was conducted where 480 ml of a culture solution was separately injected in five divided injections. The experiment was conducted for the inner diameter of the inlet tube of 3 mm, 4 mm, and 5 mm with the inner diameter of the suction tube fixed to 3 mm. Fig. 18 shows the injection volume at that time, and Table 5 shows the result of the analysis. In Table 5, logs for three times were used in No.1, and logs for five times were used in No. 2 and No. 3. In Fig. 18, the injection volume reaches the maximum value once and then settles at the final value. The time until the injection volume firstly reaches a value after being stabilized was defined as ΔT. From the above-described experiment, it was found that the flow rate was stable except the start. However, since it was conceived that use of a value closer to the value of injection end motion allows more accurate prediction, the slope for 40 ml to 60 ml was employed. The average value in Table 5 was employed as a parameter, and ΔT was determined to be 1.036 s, and the injection rate in the injection end motion was determined to be 75.7%.

[Table 5]

Table 5 Analysis result of injection work

| No. | Suction port inner diameter [mm] | Inlet port inner diameter [mm] | ΔT [s] | X [%] |
|---|---|---|---|---|
| 1 | 3 | 3 | 1.123 | 73.0 |
| 2 | 3 | 4 | 0.988 | 77.5 |
| 3 | 3 | 5 | 0.998 | 76.5 |
| Avg. | - | - | 1.036 | 75.7 |

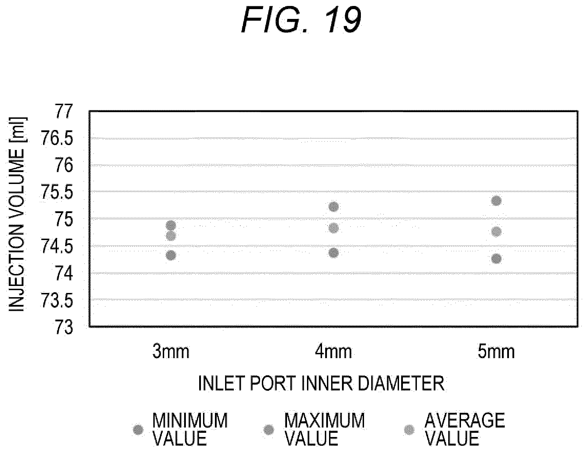

Then, operation confirmation was performed using the determined parameters. Fig. 19 and Tables 6 and 7 show the result of experiment where 480 ml of a culture solution was divided into six and 75 ml was each injected using inlet tubes (inner diameter: 3 mm, 4 mm, and 5 mm) similar to the above-described tubes. In Tables 6 and 7, logs for four times were used in the case of an inlet tube inner diameter of 3 mm, and logs for six times were used in the case of 4 mm and 5 mm. The injection work was able to be performed without dripping outside the flask or on the tip end of the inlet tube. From the results of Fig. 19 and Table 6, it was found that the same algorithm could be applied to the injection work by a robot regardless of the inner diameter of the inlet tube, and the injection work could be performed in a range of target accuracy ±2%.

[Table 6]

Table 6 Injection experimental result (injection volume/error)

| Inlet port inner diameter [mm] | Injection volume [ml] | | | Error [%] | | |
|---|---|---|---|---|---|---|
| | Min. | Max. | Avg. | Min. | Max. | Avg. |
| 3 | 74.33 | 74.88 | 74.69 | -0.9 | -0.16 | -0.42 |
| 4 | 74.38 | 75.22 | 74.83 | -0.83 | 0.29 | -0.23 |
| 5 | 74.26 | 75.33 | 74.76 | -0.98 | 0.43 | -0.33 |

[Table 7]

Table 7 Injection experimental result (flow rate/injection time)

| Inlet port inner diameter [mm] | Flow rate during injection motion [ml/s] (Avg.) | Injection time of entire injection work [s] (Avg.) |
|---|---|---|
| 3 | 2.25 | 37.02 |
| 4 | 6.57 | 15.24 |
| 5 | 10.23 | 11.16 |

Table 7 shows the flow rate of the injection motion, not including the injection start motion and the injection end motion, and the injection time of the entire injection work, including the injection start motion and the injection end motion. The flow rate increases with the increase in the inner diameter of the inlet tube, and thus the injection time decreases. Since only time during the injection motion can be shortened, approximately 5 s, corresponding to the sum of the time of the injection start motion and the injection end motion can be fixed. When the inner diameter of the inlet tube is changed from 3 mm to 4 mm, approximately 22 s can be shortened, whereas when the inner diameter of the inlet tube is changed from 4 mm to 5 mm, only approximately 4 s can be shortened. Thus, even if the inner diameter is increased to more than 5 mm, remarkable time reduction cannot be expected. In addition, it was found that the final injection volume was predicted using the flow rate during the injection motion, and therefore there was a risk that a larger flow rate caused an error.

According to the result of analysis of Example 3, injection work time per injection in the case of the manual work was approximately 70s. On the contrary, injection time in the case of using a robot was approximately 15 s even in the case of an inlet tube with an inner diameter of 4 mm. Since the culture solution is directly injected from the injection bottle in the technique conducted in this time, the suction work is unnecessary. In the case of combining a work by a robot and a manual work, it was found that work time could be reduced to half or less even when cap removal and attachment were performed by the manual work.

In the medium exchange process which is one of the important work processes in the cell processing work, to increase the application range of injection algorithm for an injection work by a robot, verification experiment was conducted by changing the constitution conditions (inner diameter) of the suction tube and the inlet tube. As a result, it was confirmed that, even in a case where the constitution conditions (inner diameter) of the suction tube and inlet tube were different and the flow rate was different (2.5 ml/s to 10.3 ml/s), the proposed injection algorithm could be applied and injection for the injection volume of 75ml could be performed in a range of target accuracy ±2%.

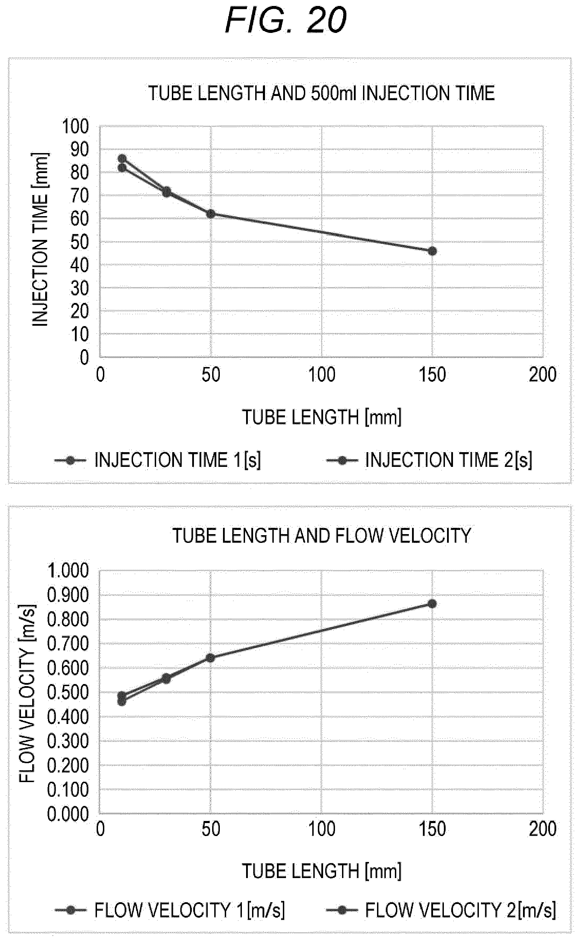

Further, the injection time in the case of changing the length of the inlet tube was measured. Table 8 and Fig. 20 show the results obtained by filling a bottle with 500 ml of a tap water, performing an injection motion by tilting the bottle by 45 deg, and measuring time until all the tap water is discharged with a stopwatch twice. It was found that there was a tendency that a longer length of the inlet tube resulted in a larger flow rate, and a shorter length of the inlet tube resulted in a smaller flow rate.

[Table 8]

Table 8 Relationship between inlet port tube length and injection time

| Inlet tube length [mm] | Injection time 1 [s] | Injection time 2 [s] |
|---|---|---|
| 10 | 86 | 82 |
| 30 | 72 | 71 |
| 50 | 62 | 62 |
| 150 | 46 | 46 |

| Inlet tube length [mm] | Flow rate 1 [m3/s] | Flow rate 2 [m3/s] |
|---|---|---|
| 10 | 5.81E − 06 | 6.10E − 06 |
| 30 | 6.94E − 06 | 7.04E − 06 |
| 50 | 8.06E − 06 | 8.06E − 06 |
| 150 | 1.09E − 05 | 1.09E − 05 |

| Inlet tube length [mm] | Flow velocity 1 [m/s] | Flow velocity 2 [m/s] |
|---|---|---|
| 10 | 0.463 | 0.485 |
| 30 | 0.553 | 0.560 |
| 50 | 0.642 | 0.642 |
| 150 | 0.865 | 0.865 |

Example 5: application to injection volume variable control algorithm

Study was conducted on whether the present invention can be applied to the cases other than the case where the injection volume is 75 ml. In a case where the injection volume is variable, the calculation section of the slope cannot be fixed. Therefore, a method was studied that employs a slope in the section excluding the injection start and injection end where the flow rate is unstable. The analysis of the results of the six time injection experiments shows that the flow rate was unstable in the section from the injection start to 5.58 ml and in the section from the injection end to 6.19 ml. Based on the analysis result, in the injection volume variable control algorithm, injection end motion start time was calculated using the slope in the section excluding 7.5 ml of the injection start and injection end.

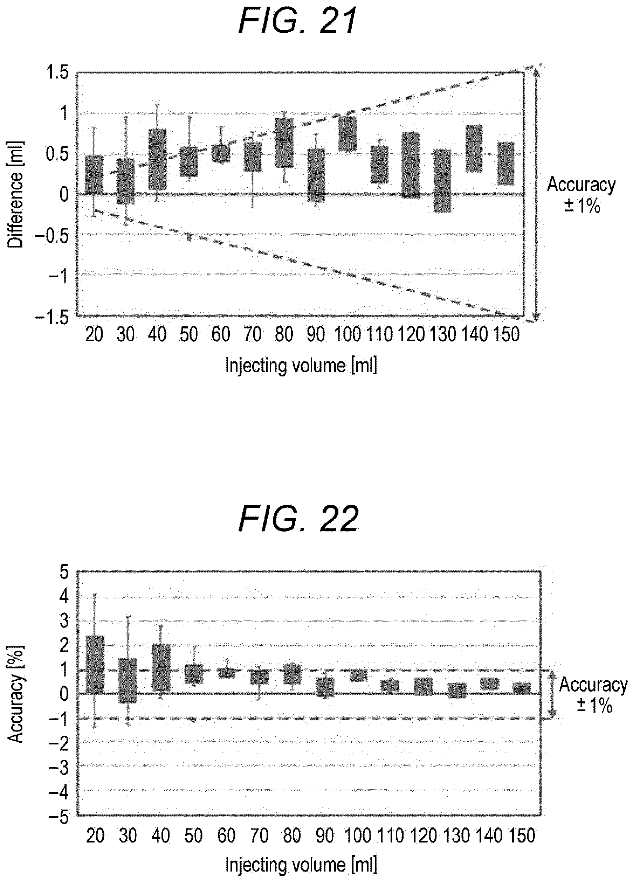

Next, operation verification was conducted using the injection volume variable control algorithm. The injection volume (injection volume) in this verification was in a range of 20 ml to 150 ml at 10 ml interval. Fig. 21 shows a difference from the target value when 450 ml of a culture solution was separately injected a plurality of times. Fig. 22 shows the injection accuracy (accuracy). Since the amount of the culture solution was fixed to 450 ml, the number of injection times was different depending on the injection volume. For example, the number of injection times in the case of the injection volume of 20 ml is 22 times, and the number of injection times in the case of the injection volume of 150 ml is three times. Fig. 21 reveals that a remarkable difference from the target value is not shown even in the case where the injection volume is different, and thus the injection volume variable control algorithm can be applied. Meanwhile, as shown in Fig. 22, with increase in the target value, the accuracy against the target value is improved.

The slope from 40 ml to 60 ml was used in Example 3, but a slope in the section excluding 7.5 ml of the injection start and injection end was used in Example 5. It was presumed that use of a value closer to the injection end motion resulted in more accurate injection. However, a difference due to the difference in the calculation section of the slope was not observed in the conditions verified in this time. Further, it was presumed that a larger injection volume results in more accurate injection, but a difference was not observed in a range of 20 ml to 150 ml. From these results, it is conceived that the flow rate during the injection motion is approximately constant. It can be therefore said that the algorithm using a slope excluding the section of the injection start and injection end is useful.

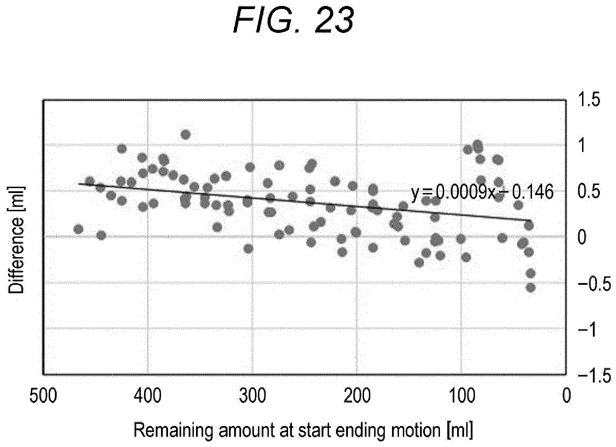

In Fig. 21, errors occur regardless of the injection volume (0.55 ml to 1.12 ml). Since 450 ml of a culture solution is continuously injected a plurality of times, the remaining amount of the culture solution in the injection bottle decreases with repeated injections. It is conceived that when the remaining amount at start of the injection end motion, the amount of the culture solution discharged during the injection end motion (motion turning the injection bottle from the attitude where the injection bottle is tilted downward by 45 deg from the horizontal direction to the attitude where the injection bottle is tilted upward by 30 deg from the horizontal direction) decreases, and thus the injection volume also decreases. Fig. 23 shows the relationship between the remaining amount at start of the injection end motion (remaining amount at start ending motion) and the difference from the target value. It can be said that when the remaining amount decreases by 450 ml, the injection volume decreases by 0.405 ml based on the linear approximation, and therefore the difference in the remaining amount at start of the injection end motion is one of the factors of an error.

As described above, it was confirmed that the injection volume variable control algorithm can be applied to the case where the injection volume is variable (20 ml to 150 ml) by setting a section excluding 7.5 ml of the injection start and end, as the calculation section of the flow rate used for estimating the injection end motion start time. It is conceived that the injection accuracy can be increased by decreasing the inner diameter of the inlet port (decreasing the flow rate), whereas the injection time becomes longer. Since the accuracy and time hold a tradeoff relationship, adjustment is required according to applications.

For example, a method for modifying injection end motion start time $\Delta T$ is shown considering that when the remaining amount at start of the injection end motion is small, the amount of culture solution discharged during the injection end motion decreases and the injection volume also decreases as described above. Specifically, in the case shown in Fig. 23, the flow rate is 6.53 [ml/s], $\Delta T$ is 0.988 [s], and X = 0.777(= 77.7%). The change (slope) in the injection volume relative to the amount of the remaining solution is 0.0009 [ml/ml], and 0.432 [ml] of injection volume decreases relative to 480 [ml]. Accordingly, the injection end motion start time is delayed with decrease in the amount of the remaining solution. That is, by delaying the injection end motion start time by $\alpha$, variation in the injection volume due to the remaining amount of the culture solution can be reduced.

Assuming that the remaining amount 480 [ml] is defined as the remaining amount at start and the remaining amount is defined as V [m], $\alpha$ can be determined from the following relational expression.

[Equation 1]

$$\alpha = \frac{0.0009}{6.53}(480 - V)$$

$\alpha$ ranges from 0 [s] at start of V = 480 [ml] to 0.0662 [s] at a remaining amount of 0 [ml]. An injection volume of 6.53 [ml/s] $\times$ 0.0662 [s] = 0.432 [ml] can be increased by delaying the injection end motion start time by 0.066 [s]. This corresponds to the amount of decrease in the injection volume 480 [ml] $\times$ 0.0009 [ml/ml] = 0.432 [ml]. Similarly, it becomes possible to reduce variation in the injection volume due to the remaining amount of the culture solution by modifying X. Assuming that the remaining amount 480 [ml] is defined as the remaining amount at start and the remaining amount is defined as V [m], X can be determined from the following relational expression.

[Equation 2]

$$X = \frac{0.0670}{480}V + 0.710$$

X ranges from 0.777(= 77.7%) at start time of V = 480 [ml] to 0.710(=71.0%) at a remaining amount of 0 [ml]. The injection volume at start of V = 480 [ml] after starting the injection end motion is 6.53 [ml/s] $\times$ 0.988 [s] $\times$ 0.777 = 5.013 [ml], and the injection volume at a remaining amount of 0 [ml] is 6.53 [ml/s] $\times$ 0.988 [s] $\times$ 0.710 = 4.581 [ml]. Accordingly, in the latter injection volume, the injection volume after starting the injection end motion decreases by 5.013 [ml] - 4.581 [ml] = 0.432 [ml]. That is, the injection end motion start time is delayed by 0.432 [ml]/6.53 [ml/s] = 0.066 [s].

In the above description, a case where the remaining amount at start, that is, the maximum remaining amount ($V_{max}$) of the injection bottle is 480 [ml] has been described. It is also possible to perform dispense of 75 ml $\times$ 12 times to 13 times using a $V_{max}$ = 1,000 ml bottle. In this case, when $V_{max}$ = 480 [ml] in the above Equation 2 is $V_{max}$ = 1000 [ml], a change in a range of 0.710 to 0.777 occurs between $V_{max}$ =1000 (maximum remaining amount) to 0 (minimum

remaining amount). $V_{max}$ is not particularly limited, and for example, may be 200 to 20,000 [ml], preferably 250 to 10,000 [ml], even more preferably 300 to 8,000 [ml], particularly preferably 350 to 5,000 [ml], and most preferably 400 to 2,000 [ml].

In the present invention, the phrase "delaying start time of the injection end control (motion)" means delaying start time of the injection end control (motion) by varying (decreasing) X by approximately 0 to 10 [%] from the maximum remaining amount ($V_{max}$) to the minimum remaining amount ($V_{min}$) of the injection bottle, and X is determined from the following relational expression.

[Equation 3]

$$ X = \frac{X_d}{V_{max}} + V_f $$

Here, $V_{max}$: maximum remaining amount (volume) of injection bottle, Xd: amount of change in X, and Vf: final X value

## Claims

1. A robot system for injecting liquid by rotating an injection container in which the liquid is accommodated and an injection volume of the liquid is constant around an axis vertical to a long axis of the injection container, wherein the robot system executes
an injection start control to rotate the injection container around a predetermined axis,
an injection control to stop rotation for a predetermined time and inject the liquid, and
an injection end control to reversely rotate the injection container around the predetermined axis, and
the predetermined time is calculated based on an injection flow rate Q [ml/s] measured in real time.

2. The system according to claim 1, wherein the predetermined time is further calculated based on time $\Delta T$ until liquid is not injected in reverse rotation.

3. The system according to claim 1 or 2, wherein the predetermined time is further calculated based on a final injection ratio X% of an injection volume in a case where an injection end step is performed relative to an injection volume in a case where the injection end step is not performed.

4. The system according to any one of claims 1 to 3, wherein a device is mounted on the injection container, the device including a cap that can be detachably attached to the injection container, an inlet tube that can be fitted in a first through hole provided in the cap, and a suction tube that can be fitted in a second through hole provided in the cap, and the predetermined axis is set at a tip end of the inlet tube.

5. The system according to claim 4, wherein the system is configured so that, in a state in which the device is mounted on the injection container, a lower end of the suction tube protruding from the cap into the injection container is disposed close to the cap.

6. The system according to any one of claims 1 to 5, wherein the system is configured to delay a start time of the injection end control with decrease in a remaining amount of the liquid in the injection container.

7. A method for injecting liquid by rotating an injection container in which the liquid is accommodated and an injection volume of the liquid is constant around an axis vertical to a long axis of the injection container, the method comprising:

    an injection start step of rotating the injection container around a predetermined axis;
    an injection step of stopping rotation for a predetermined time and injecting the liquid; and
    an injection end step of reversely rotating the injection container around the predetermined axis, wherein the predetermined time is calculated based on an injection flow rate Q [ml/s] measured in real time.

8. A program for controlling a robot for injecting liquid by rotating an injection container in which the liquid is accommodated and an injection volume of the liquid is constant around an axis vertical to a long axis of the injection container, wherein the program causes a computer to execute

an injection start control to rotate the injection container around a predetermined axis,

an injection control to stop rotation for a predetermined time and inject the liquid, and

an injection end control to reversely rotate the injection container around the predetermined axis, and

the predetermined time is calculated based on an injection flow rate Q [ml/s] measured in real time.

## FIG. 1

## FIG. 2

*FIG. 3*

(A)

(B)

*FIG. 4*

## FIG. 5

## FIG. 6

## FIG. 7

```
┌──────────────────────────┐
│ LIQUID-DISCARDING PROCESS │
└──────────────────────────┘
              │
              ▼
┌──────────────────────┐      ┌──────────────┐
│   INJECTION PROCESS   │ ───▶ │  CAP REMOVAL  │
└──────────────────────┘      └──────────────┘
                                      │
                                      ▼
                              ┌──────────────┐      ┌────────────────────────┐
                              │ INJECTION WORK │ ──▶ │  INJECTION START MOTION │
                              └──────────────┘      └────────────────────────┘
                                      │                         │
                                      ▼                         ▼
                              ┌──────────────┐      ┌────────────────────────┐
                              │ CAP ATTACHMENT │     │    INJECTION MOTION     │
                              └──────────────┘      └────────────────────────┘
                                                                │
                                                                ▼
                                                    ┌────────────────────────┐
                                                    │   INJECTION END MOTION  │
                                                    └────────────────────────┘
```

## FIG. 8

## *FIG. 9*

## *FIG. 10*

# FIG. 11

EXTRACTION OF PARAMETER BY EXPERIMENT

PERFORM EXPERIMENT (5 times×3 sets)
(VOLUME AT START OF INJECTION END MOTION=70ml)

↓

CALCULATE TIME Δt AND INJECTION RATIO X DURING
INJECTION END MOTION FROM EXPERIMENTAL DATA

⇩

VALIDATION TEST USING PARAMETER (6 times×3 sets)

CALCULATE INCLINATION FROM 40ml TO 60ml

↓

CALCULATE VOLUME AT START OF INJECTION END MOTION
USING Δt AND INJECTION RATIO X IN INJECTION END MOTION

↓

START INJECTION END MOTION USING CALCULATION RESULT

# FIG. 12

Vmax

START
↓
INJECTION START MOTION
↓
INJECTION VOLUME>60ml ──N
↓ Y
CALCULATE INJECTION FLOW
RATE Q[m/s] FROM INJECTION
TIME OF 40ml TO 60ml
↓ Q

ΔT: FINAL INJECTION
VOLUME REACH TIME →
CALCULATE INJECTION VOLUME
(VOLUME CORRESPONDING TO 100%)
Vmax[ml] IN CASE WHERE INJECTION
END MOTION IS NOT PERFORMED

CALCULATE INJECTION END
MOTION INJECTION Vx[ml] ← X: FINAL
INJECTION RATIO
↓ Vx
CALCULATE INJECTION
VOLUME AT START OF
INJECTION END MOTION
(Ve=Vt-Vx) ← Vt: TARGET INJECTION
VOLUME 75[ml]
↓ Ve
INJECTION VOLUME
>Ve[ml] ──N
↓ Y
INJECTION END MOTION START
↓
END

25

# FIG. 13

(1) Overall view

(2) Enlarged view

# FIG. 14

## FIG. 15

SUCTION PORT
Φ2, 3, 4

INLET PORT
Φ3, 4, 5

51.5  23  25

14  15

21.5  50  25

φ49.4

SUCTION PORT    INLET PORT

## FIG. 16

FLOW RATE [ml/s]

TIME [s]

——2mm ——3mm ——4mm

## FIG. 17

FLOW RATE [ml/s]

TIME [s]

——3mm ——4mm ——5mm

# FIG. 18

(1) OVERALL VIEW

(2) ENLARGED VIEW (INLET PORT INNER DIAMETER=4mm)

# FIG. 19

# FIG. 20

TUBE LENGTH AND 500ml INJECTION TIME

INJECTION TIME [mm] vs TUBE LENGTH [mm]

INJECTION TIME 1[s]    INJECTION TIME 2[s]

TUBE LENGTH AND FLOW VELOCITY

FLOW VELOCITY [m/s] vs TUBE LENGTH [mm]

FLOW VELOCITY 1[m/s]    FLOW VELOCITY 2[m/s]

## FIG. 21

## FIG. 22

# FIG. 23

## INTERNATIONAL SEARCH REPORT

| International application No. |
| --- |
| PCT/JP2019/021642 |

**A. CLASSIFICATION OF SUBJECT MATTER**
Int.Cl. C12M1/00(2006.01)i, C12M1/10(2006.01)i

According to International Patent Classification (IPC) or to both national classification and IPC

**B. FIELDS SEARCHED**

Minimum documentation searched (classification system followed by classification symbols)
Int.Cl. C12M1/00, C12M1/10

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN), WPIDS/WPIX (STN)

**C. DOCUMENTS CONSIDERED TO BE RELEVANT**

| Category* | Citation of document, with indication, where appropriate, of the relevant passages | Relevant to claim No. |
| --- | --- | --- |
| A | 野々山良介他，細胞処理作業の効率化システムに関する研究（システムコンセプトと培地交換プロセスにおけるロボットによる廃液作業の効率化），日本機械学会論文集, 07 March 2018, vol. 84, no. 859, DOI: 10.1299/transjsme.17-00497, pp. 1-13, 3・1, 3・3・1, 6, (NONOYAMA, Ryosuke et al., "Study on cell culture processing system to improve task efficiency (System concept and efficiency improvement of the discarding task using a robot in a media change process)", Transactions of the JSME) | 1-8 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 23 August 2019 (23.08.2019) | 03 September 2019 (03.09.2019) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/021642 |

C (Continuation). DOCUMENTS CONSIDERED TO BE RELEVANT

| Category* | Citation of document, with indication, where appropriate, of the relevant passages | Relevant to claim No. |
| --- | --- | --- |
| P,X | 野々山良介他，細胞処理作業の効率化システムに関する研究（培地交換プロセスにおけるロボットによる注液作業の効率化），日本機械学会論文集，20 May 2019, vol. 84, no. 874, DOI: 10.1299/transjsme.18-00421, pp. 1-13, entire text, (NONOYAMA, Ryosuke et al., "Study on cell culture processing system to improve task efficiency (Efficiency improvement of the injecting task using a robot in a media change process)", Transactions of the JSME) | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016104666 A